(19) Europäisches Patentamt
European Patent Office
Office européen des brevets

(11) Veröffentlichungsnummer: **0 005 504**
**A1**

(12) # EUROPÄISCHE PATENTANMELDUNG

(21) Anmeldenummer: **79101412.9**

(51) Int. Cl.$^2$: **C 07 D 239/10**

(22) Anmeldetag: **09.05.79**

(30) Priorität: **13.05.78 DE 2821086**

(43) Veröffentlichungstag der Anmeldung:
**28.11.79 Patentblatt 79/24**

(84) Benannte Vertragsstaaten:
**BE CH DE FR GB IT**

(71) Anmelder: **BASF Aktiengesellschaft**
**Carl-Bosch-Strasse 38**
**D-6700 Ludwigshafen(DE)**

(72) Erfinder: **Petersen, Harro, Dr.**
**Kalmitstrasse 23**
**D-6710 Frankenthal(DE)**

(54) **Verfahren zur Herstellung von 5,5-disubstituierten 4-Ureido-hexahydropyrimidinen.**

(57) Herstellung von 5,5-disubstituierten 4-Ureido-hexahydropyrimidinen durch Umsetzung von Harnstoffen mit Aldehyden bei Temperaturen von zuerst unterhalb 110°C und dann oberhalb 110°C.

Die nach dem Verfahren der Erfindung herstellbaren 5,5-di-substituierten 4-Ureido-hexahydropyrimidine sind wertvolle Ausgangsstoffe für die Herstellung von Hilfsmitteln in der Textilchemie, Lackharzchemie und Holzwerkstoffindustrie.

EP 0 005 504 A1

# Verfahren zur Herstellung von 5,5-disubstituierten 4-Ureido-hexahydropyrimidinen

Die Erfindung betrifft ein neues Verfahren zur Herstellung von 5,5-disubstituierten 4-Ureido-hexahydropyrimidinen durch Umsetzung von Harnstoffen mit Aldehyden bei Temperaturen von zuerst unterhalb 110°C und dann oberhalb 110°C.

Es ist bekannt, daß man durch Cyclokondensation von Harnstoffen mit einem Mol Formaldehyd und einem oder 2 Mol eines CH-aciden Aldehyds wie Isobutyraldehyd in Gegenwart von Säuren zu 4-Hydroxy-2-oxo-hexahydropyrimidinen gelangt, z.B.:

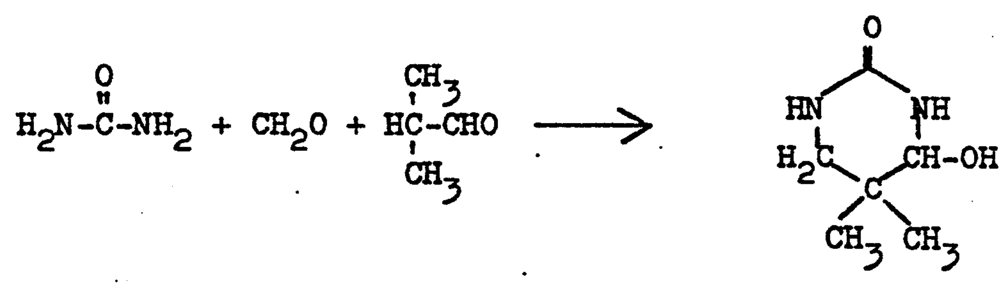

(Ynthesis, Band 1973, Seiten 243 bis 292, insbesondere Seite 262 und 263). Reaktionstemperaturen unterhalb 90°C werden angegeben. Diese Umsetzungen verlaufen mit befriedigenden Ausbeuten nur in den Fällen in denen ein CH-aci-

WB/Fe

der Aldehyd eingesetzt wird, der in α-Stellung zwei Substituenten aufweist. Setzt man CH-acide Aldehyde ein, die in α-Stellung keinen (Acetaldehyd) oder nur einen Substituenten (n-Aldehyde) aufweisen, so erhält man bei der sauren Kondensation mit Harnstoffen 2-Oxo(thiono)-4-ureido-hexahydropyrimidine (IV) oder Dioxo(thiono)-decahydropyrimidopyrimidine (V):

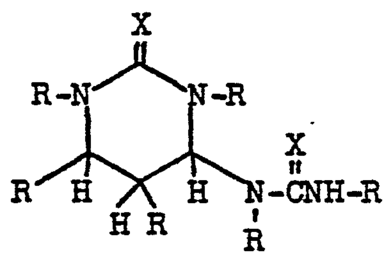
IV

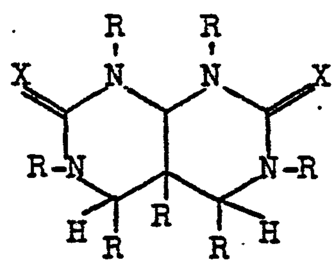
V

X = O, S
R = H, Alkyl

Bei Einsatz derartiger CH-acider Aldehyde bleibt die Reaktion nicht bei den 4-Hydroxy-Derivaten stehen (Synthesis Band 1973, Seiten 261 bis 280).

Ferner lassen sich 2-Oxo(thiono)-4-hydroxy-(alkoxy)-hexahydropyrimidine, die in 5-Stellung keinen oder nur einen Substituenten aufweisen, durch Umsetzung von Harnstoffen mit α,ß-ungesättigten Aldehyden in Gegenwart von Säure herstellen, z.B.:

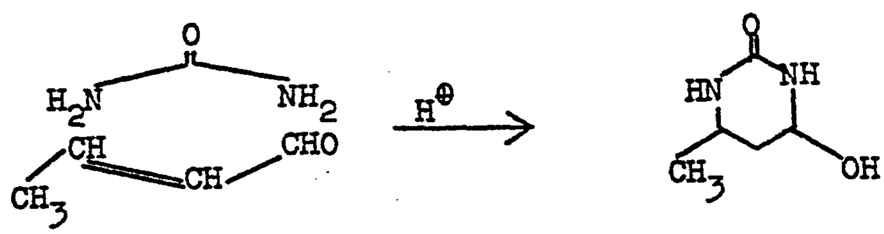

Die so hergestellten 4-Hydroxy-Derivate reagieren mit Harnstoffen zu den 4-Ureido-Derivaten (Synthesis, loc. cit., Seite 264). Die Synthese der in 5-Stellung unsubstituierten oder monosubstituierten 2-Oxo(thiono)-4-hydroxy-(alkoxy)-hexahydropyrimidine ist auf diesem Syntheseweg umständlich und verläuft mit unbefriedigenden Ausbeuten.

In den Monatsheften für Chemie, Band 96 (1965), Seiten 1 950 bis 1 966 (insbesondere Seite 1 957) wird beschrieben, daß die Substitution der Hydroxygruppe in 4-Stellung durch Nucleophile erschwert wird, wenn in 5-Stellung ein Substituent steht. Bei zwei Substituenten in 5-Stellung läßt sich eine Substitution der OH-Gruppe in Stellung 4 durch Harnstoffe unter den bisher bekannten Bedingungen nicht mehr durchführen. So ist es auch verständlich, daß es unter den üblichen Bedingungen der Säurekatalyse bisher nicht möglich war, in 5-Stellung disubstituierte 4-Hydroxyhexahydropyrimidine mit Harnstoffen zu 5,5-disubstituierten 2-Oxo(thiono)-4-ureido-hexahydropyrimidinen umzusetzen.

Es wurde nun gefunden, daß man 5,5-disubstituierte 4-Ureido-hexahydropyrimidine der Formel

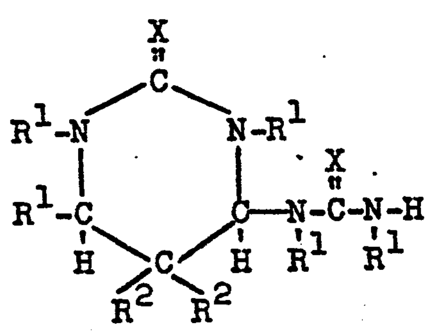

I,

worin die einzelnen Reste $R^1$ und $R^2$ gleich oder verschieden sein können und jeweils einen aliphatischen, cycloaliphatischen, aliphatischaromatischen, araliphatischen oder aromatischen Rest bedeuten, darüber hinaus auch die Reste $R^1$ jeweils ein Wasserstoffatom bezeichnen können, die einzelnen Reste X jeweils ein Sauerstoffatom oder ein Schwefelatom bedeuten, vorteilhaft erhält, wenn man Harnstoffe der Formel

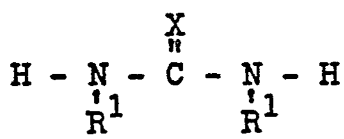 II,

worin $R^1$ und X die vorgenannte Bedeutung besitzen,

a) mit Aldehyden der Formel

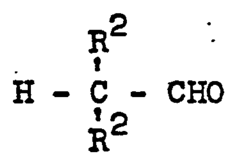 III,

worin $R^2$ die vorgenannte Bedeutung besitzt, oder

b) mit Formaldehyd und Aldehyden der Formel III oder

c) mit Aldehyden III und Harnstoffen der Formel

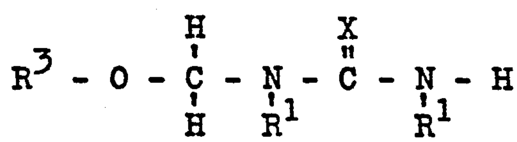 IV,

worin $R^1$ und X die vorgenannte Bedeutung besitzen, und $R^3$ ein Wasserstoffatom oder einen aliphatischen Rest bezeichnet,

in einem ersten Schritt bei einer Temperatur unterhalb von $110^{o}C$ und anschließend in einem zweiten Schritt bei einer Temperatur oberhalb von $110^{o}C$ in Gegenwart einer Säure umsetzt.

Die Umsetzung kann für den Fall der Verwendung von Harnstoff, N,N'-Dimethylharnstoff, Isobutyraldehyd, Formaldehyd und N,N'-Dimethyl-N-methoxymethylharnstoff durch die folgenden Formeln wiedergegeben werden:

a)

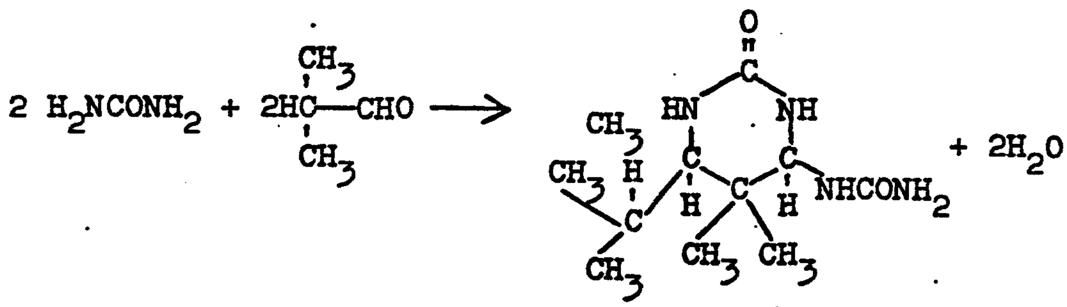

b)

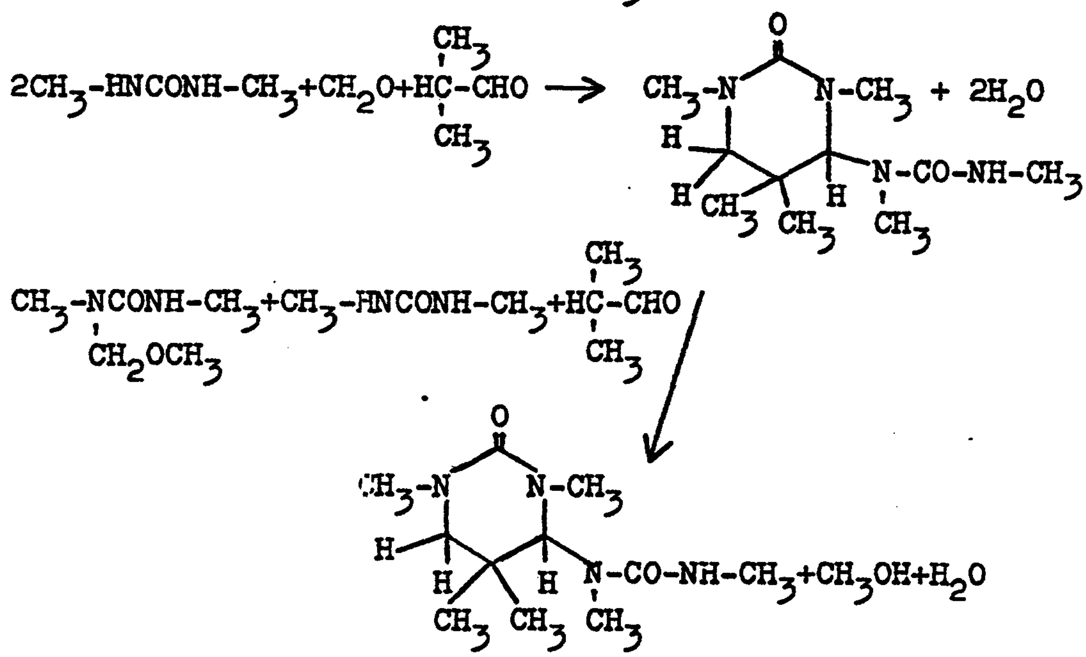

c)

Im Hinblick auf den Stand der Technik liefert das Verfahren nach der Erfindung auf einfacherem und wirtschaftlicherem Wege eine große Zahl von 5,5-disubstituierten 4-Ureidohexahydropyrimidinen in guter Ausbeute und Reinheit. Alle diese vorteilhaften Ergebnisse sind im Hinblick auf den Stand der Technik überraschend. Man hätte angesichts der hohen Reaktionstemperaturen und des sauren Reaktionsmediums im zweiten Verfahrensschritt erhöhte Zersetzung des Reaktionsgemisches und somit die Bildung heterogener Gemische von Neben- und Zersetzungsprodukten und eine wesentlich geringere Ausbeute an Endstoff bzw. eine Blockierung der erfindungsgemäßen Reaktion erwarten müssen.

Die Ausgangsstoffe II und III können in stöchiometrischer Menge oder im Überschuß jeder Komponente zur anderen, vorzugsweise im Falle a) in einem Verhältnis von 1 bis 1,5, insbesondere 1 bis 1,1 Mol Ausgangsstoff III je Mol Ausgangsstoff II, im Falle b) in einem Verhältnis von 0,5 bis 0,75, insbesondere 0,5 bis 0,55 Mol Ausgangsstoff III je Mol Ausgangsstoff II bzw. ineinem Verhältnis von 0,5 bis 0,75, insbesondere 0,5 bis 0,55 Mol Formaldehyd je Mol Ausgangsstoff II, im Falle c) in einem Verhältnis von 0,5 bis 0,75, insbesondere 0,5 bis 0,55 Mol Ausgangsstoff III je Mol Ausgangsstoff II bzw. in einem Verhältnis von 0,5 bis 0,75, insbesondere 0,5 bis 0,55 Mol Ausgangsstoff IV je Mol Ausgangsstoff II, umgesetzt werden. Bevorzugte Ausgangsstoffe II, III und IV und dementsprechend bevorzugte Endstoffe I sind solche, in deren Formeln die einzelnen Reste $R^1$ und $R^2$ gleich oder verschieden sein können und jeweils einen Alkylrest mit 1 bis 18, insbesondere 1 bis 7 Kohlenstoffatomen, einen Cyclohexylrest, einen Alkylarylrest oder Aralkyrest mit 7 bis 12 Kohlenstoffatomen oder einen Phenylrest bedeuten, darüber hinaus auch die Reste $R^1$ jeweils ein Wasserstoffatom bezeichnen können, die einzelnen Reste X gleich oder verschieden sein können

und jeweils ein Sauerstoffatom oder eine Schwefelatom bedeuten, $R^3$ ein Wasserstoffatom oder einen Alkylrest mit 1 bis 7 Kohlenstoffatomen bezeichnet. Die vorgenannten Reste und Ringe können noch durch unter den Reaktionsbedingungen inerte Gruppen, z.B. Alkylgruppen mit 1 bis 4 Kohlenstoffatomen, substituiert sein. Geeignete Harnstoffe II sind unsubstituierte, monosubstituierte oder symmetrisch disubstituierte Harnstoffe. Formaldehyd kann in Gestalt von Paraformaldehyd oder im Gemisch mit Wasser, z.B. in Gestalt einer 35- bis 50-gewichtsprozentigen Lösung, verwendet werden.

Geeignete Harnstoffe III sind beispielsweise: Harnstoff, Thioharnstoff, Monomethylharnstoff, Monophenylharnstoff, Monostearylharnstoff, o-, m- bzw. p-Methylphenylharnstoff, o-, m- bzw. p-Äthylphenylharnstoff, Monobenzyl-, Monocyclohexyl-, Monoäthyl-, Monopropyl-, Monoisopropyl-, Monobutyl-, Mono-sek.-butyl-, Mono-tert.-butyl-, Mono-n-butylharnstoff; symmetrischer Dimethyl-, Diphenyl-, Dibenzyl-, Dicyclohexyl-, Diäthyl-, Dipropyl-, Diisopropyl-, Dibutyl-, Di-sek.-butyl-, Diisobutyl-, Di-tert.-butyl-harnstoff; entsprechend substituierte Thioharnstoffe.

Geeignete Harnstoffe IV sind beispielsweise die vorgenannten unsubstituierten bzw. substituierten Harnstoffe und Thioharnstoffe, die noch an einem Stickstoffatom zusätzlich durch eine Hydroxy-, Methoxy-, Äthoxy-, Propoxy-, Isopropoxy-, Butoxy-, Isobutoxy-, sek.-Butoxy-, tert.-Butoxy-methylgruppe substituiert sind.

Es können z.B. folgende Aldehyde als Ausgangsstoffe III verwendet werden: 2-Äthyl-2-hexyl-, 2-Methyl-2-n-heptyl-, 2-Äthyl-2-pentyl-, 2-Methyl-2-pentyl-, 2-Methyl-2-n-butyl-, 2-Propyl-2-pentyl-, 2-Butyl-2-pentyl-, 2-Äthyl-2-heptyl, 2-Propyl-2-heptyl-, 2-Butyl-2-heptyl-, 2-Pentyl-2-

heptyl-, 2-Methyl-2-hexyl-, 2-Propyl-2-hexyl-, 2-Butyl-2-hexyl-, 2-Isopropyl-2-hexyl-, 2-Isobutyl-2-hexyl-, 2,2-Diphenyl-, 2,2-Dibenzyl-, 2-Phenyl-2-äthyl-, 2,2-Dicyclohexyl-, 2,2-Di-(p-methylphenyl)-, 2,2-Di-(o-methylphenyl)-, 2,2-Di-(m-methylphenyl)-, 2-Methyl-2-benzyl-, 2-Di-(äthyl)-, 2-Di-(n-propyl)-, 2-Di-(isopropyl)-, 2-Di-(n-butyl)-, 2-Di-(isobutyl)-, 2-Di-(sek.-butyl)-, 2-Di-(tert.-butyl)-, 2-Di-(pentyl)-, 2-Di-(pentyl)-(2)-, 2-Di-(pentyl)-(3), 2-Di-(n-Hexyl)-, 2-Di-(n-heptyl)-, 2-Di-(n-octyl)-, 2-Di-(n-nonyl)-, 2-Di-(n-decyl)-, 2-Di-(2-äthylhexyl)-acetaldehyd; bevorzugt sind Isobutyraldehyd, 2-Äthylhexanal, 2-Phenylpropanal.

Die Umsetzung wird im ersten Schritt der Reaktion bei einer Temperatur unterhalb von 110°C, vorzugsweise zwischen 30 und 110°C, insbesondere von 70 bis 100°C; im zweiten Schritt der Reaktion bei einer Temperatur oberhalb von 110°C, zweckmäßig zwischen 110 und 150°C, vorzugsweise von 120 bis 145°C, insbesondere von 125 bis 140°C, drucklos oder unter Druck, kontinuierlich oder diskontinuierlich durchgeführt. Man kann ohne Lösungsmittel umsetzen; zweckmäßig verwendet man aber unter den Reaktionsbedingungen inerte Lösungsmittel, insbesondere mit Siedepunkten über 100°C. Bei Flüssigkeiten mit Siedepunkten unter 100°C wird die Reaktion zweckmäßig in geschlossenen Gefäßen unter Druck durchgeführt. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Dimethylformamid; und entsprechende Gemische. Bevorzugte Lösungsmittel sind Toluol und Xylole. Es ist nicht erforderlich, daß die Ausgangsstoffe und Endstoffe in diesen Lösungsmitteln löslich sind. Die Unlöslichkeit der Endstoffe ermöglicht eine besonders leichte Aufarbeitung. In der Regel wird das gebildete Reaktionswasser bzw. das gebildete Alkanol abgeführt, vorteilhaft durch fraktionier-

te oder azeotrope Destillation. Zweckmäßig verwendet man das Lösungsmittel in einer Menge von 400 bis 10 000 Gewichtsprozent, vorzugsweise von 400 bis 1 000 Gewichtsprozent, bezogen auf Ausgangsstoff II. Die Umsetzung kann zweckmäßig in Abwesenheit von Lösungsmitteln durchgeführt werden, wenn eine oder beide Ausgangskomponenten oder der Endstoff flüssig oder bei der Reaktionstemperatur schmelzbar sind. Die Reaktion wird im ersten Schritt, im allgemeinen aber in beiden Verfahrensschritten in Gegenwart von Säure, vorteilhaft in einer Menge von 0,005 bis 0,1, insbesondere von 0,01 bis 0,05 Äquivalenten Säure, bezogen auf 1 Mol Ausgangsstoff II, durchgeführt. Es können anorganische oder organische Säuren verwendet werden. Anstelle einbasischer Säuren können auch äquivalente Mengen mehrbasischer Säuren zur Anwendung gelangen. Beispielsweise sind folgende Säuren geeignet: Chlorwasserstoff, Schwefelsäure, Phosphorsäure, Salpetersäure; Sulfonsäuren wie Benzol- und p-Toluolsulfonsäure; aliphatische Carbonsäuren wie Oxalsäure; aromatische Carbonsäuren wie Benzoesäure, Phthalsäure oder entsprechende Gemische. Die Säuren können in konzentrierter Form, im Gemisch miteinander und/oder mit einem Lösungsmittel, insbesondere Wasser, angewendet werden. Bei verdünnten, wäßrigen Säuren sind 10- bis 80-gewichtsprozentige Säuren, z.B. 10- bis 30-gewichtsprozentige Salzsäure, 10- bis 75-gewichtsprozentige Schwefelsäure oder 10- bis 50-gewichtsprozentige Oxalsäure, vorteilhaft. Bevorzugt sind Salzsäure, Schwefelsäure, p-Toluolsulfonsäure, Oxalsäure, Phthalsäure. der pH der Umsetzung beträgt vorzugsweise von 1 bis 3, insbesondere von 1 bis 2.

Die Reaktion kann wie folgt durchgeführt werden: Ein Gemisch der Ausgangsstoffe, im Falle a) der Ausgangsstoffe II und III, im Falle b) der Ausgangsstoffe II, III und Formaldehyd, im Falle c) der Ausgangsstoffe II, III und

IV, zusammen mit Säure und zweckmäßig Lösungsmittel wird im ersten Reaktionsschritt während 0,25 bis 1,5 Stunden bei der Reaktionstemperatur des ersten Schrittes gehalten. Nun wird die Temperatur auf die des zweiten Reaktionsschrittes erhöht und zweckmäßig während und insbesondere nach Erreichung der Temperatur des zweiten Schrittes das gebildete Reaktionswasser bzw. das abgespaltene Alkanol (im Falle c)) abgetrennt. Der zweite Schritt wird in der Regel während 0,25 bis 2,5 Stunden durchgeführt. Dann wird der Endstoff aus dem Gemisch in üblicher Weise, z.B. durch Destillation oder Filtration, abgetrennt.

Die nach dem Verfahren der Erfindung herstellbaren 5,5-disubstituierten 4-Ureido-hexahydropyrimidine sind wertvolle Ausgangsstoffe für die Herstellung von Hilfsmitteln in der Textilchemie, Lackharzchemie und Holzwerkstoffindustrie. Ihre N-Methylol- und N-Alkoxymethylverbindungen sind als reaktive Vernetzer in der Textilchemie, Lackharzschemie und Holzwerkstoffindustrie vorteilhaft verwendbar. Die 2-Oxo-4-ureido-hexahydropyrimidine sind wertvolle Stickstoff-Depotdüngemittel und können z.B. auf die in den deutschen Patentschriften 1 081 482, 1 244 207, 1 223 843 oder 1 467 378 beschriebene Weise verwendet werden. So ist z.B. das 2-Oxo-4-ureido-5,5-dimethyl-6-isopropyl-hexahydropyrimidin in Wasser schwer löslich. Bei der Einbringung in den Boden werden insbesondere empfindliche Pflanzen, z.B.im Wachstumsstadium, und Keimlinge nicht durch den von Mineraldüngemitteln her bekannten Salzeffekt geschädigt. Für die Düngung mit den schwerlöslichen oder unlöslichen Endstoffen I werden je Hektar Weide- und Rasenflächen zweckmäßig 100 bis 300 kg (definiert als kg Rein-Stickstoff) eingesetzt.

Die in den Beispielen genannten Teile bedeuten Gewichtsteile.

Beispiel 1

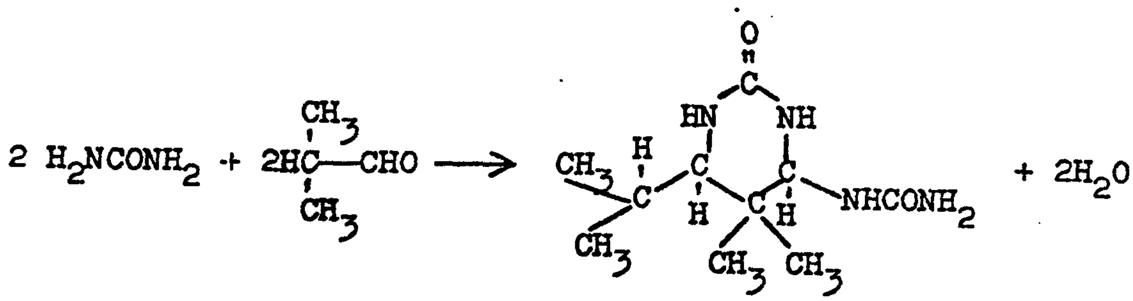

In einer Rührapparatur mit Rührer, Rückflußkühler, Heizung und zuschaltbarem Wasserabscheider werden 300 Teile Harnstoff zusammen mit 360 Teilen Isobutyraldehyd, 1 500 Teilen Xylol und 50 Teilen einer 50-gewichtsprozentigen Schwefelsäure zwei Stunden bei Rückflußtemperatur erwärmt. Die Rückflußtemperatur steigt von 80°C auf 95°C an. Danach wird der Wasserabscheider zugeschaltet und das Reaktionswasser sowie das über die Schwefelsäure eingebrachte Wasser durch azeotrope Destillation bei 125°C innerhalb einer Stunde abgetrennt (115 Teile). Der Endstoff wird nach dem Abkühlen durch Filtration abgetrennt. Nach Trocknung werden 530 Teile 2-Oxo-4-ureido-5,5-dimethyl-6-isopropylhexahydropyrimidin erhalten. Das entspricht einer Ausbeute von 97 % der Theorie. Schmelzpunkt 208°C.

Beispiel 2

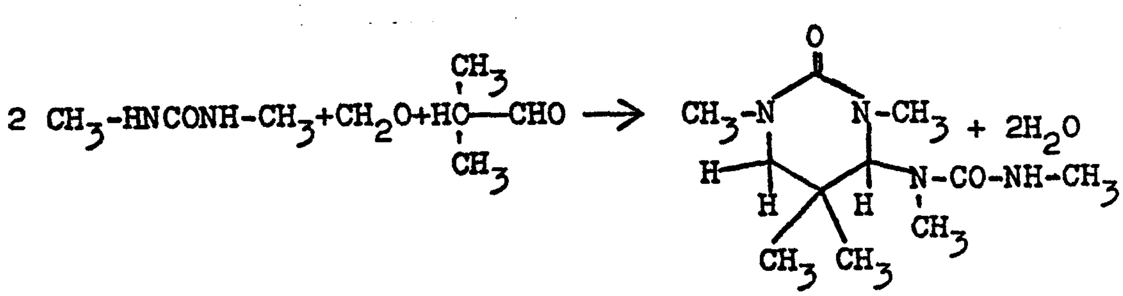

Analog Beispiel 1 wird das Gemisch aus 440 Teilen symmetrischem Dimethylharnstoff mit 75 Teilen Paraformaldehyd, 180 Teilen Isobutyraldehyd, 30 Teilen einer 98-gewichtsprozentigen Schwefelsäure und 700 Teilen Xylol eine Stunde bei Rückflußtemperatur (90°C) erwärmt. Danach wird der Wasserabscheider zugeschaltet und der zweite Reaktionsschritt mit Wasserauskreisung in zwei Stunden bei 125°C durchgeführt. 86 Teile Wasser werden ausgekreist. Xylol wird anschließend unter vermindertem Druck abgedampft. Es werden 579 Teile 2-Oxo-4-(N,N'-dimethylureido)-1,3,5,5-tetramethyl-hexahydropyrimidin vom Fp 102 bis 104°C erhalten. Das entspricht einer Ausbeute von 96 % der Theorie.

Beispiel 3

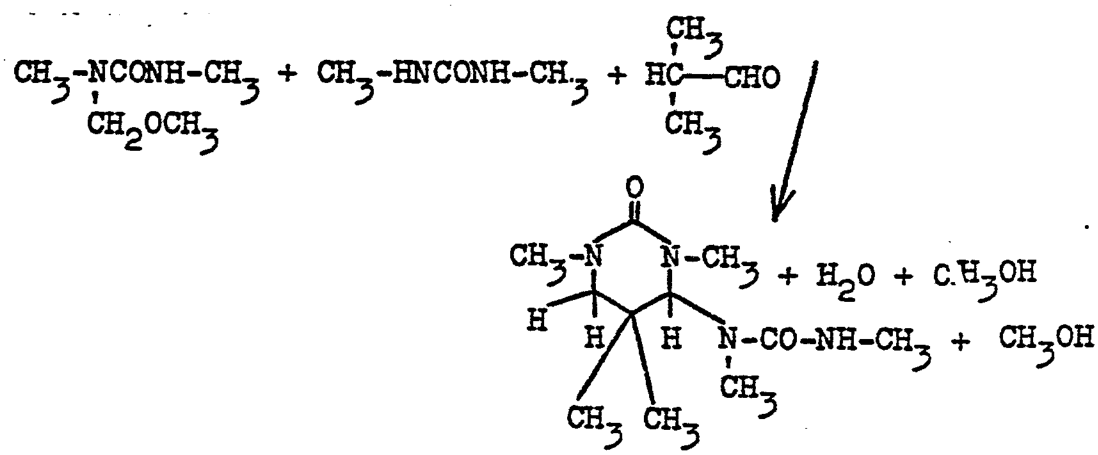

Analog Beispiel 1 wird das Gemisch von 66 Teilen N,N'-Dimethyl-N-methoxymethylharnstoff, 44 Teilen N,N'-Dimethylharnstoff, 36 Teilen Isobutyraldehyd und 400 Teilen Xylol eine halbe Stunde auf Rückflußtemperatur (90°C) erhitzt. Dann wird das gebildete Methanol während 30 Minuten bei einer Sumpftemperatur von 125°C abdestilliert. Anschließend wird das Xylol abdestilliert. Es werden 105 Teile 2-Oxo-4-(N,N'-dimethylureido)-1,3,5,5-tetramethyl-hexahydropyrimidin (97 % der Theorie) vom Fp 101 bis 103°C erhalten.

Patentanspruch

Verfahren zur Herstellung von 5,5-disubstituierten 4-Ureido-hexahydropyrimidinen der Formel

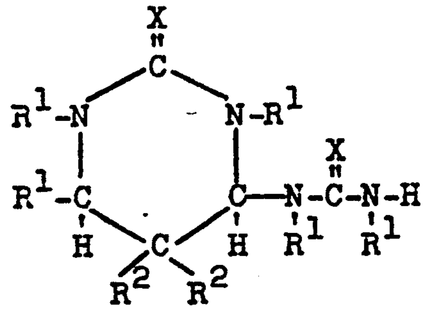

I,

worin die einzelnen Reste $R^1$ und $R^2$ gleich oder verschieden sein können und jeweils einen aliphatischen, cycloaliphatischen, aliphatischaromatischen, araliphatischen oder aromatischen Rest bedeuten, darüber hinaus auch die Reste $R^1$ jeweils ein Wasserstoffatom bezeichnen können, die einzelnen Reste X jeweils ein Sauerstoffatom oder ein Schwefelatom bedeuten, dadurch gekennzeichnet, daß man Harnstoffe der Formel

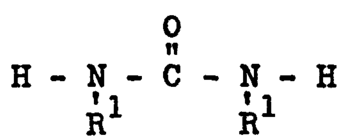

II,

worin $R^1$ und X die vorgenannte Bedeutung besitzen,

a) mit Aldehyden der Formel

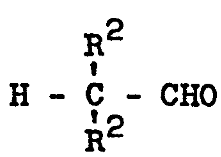

III,

worin $R^2$ die vorgenannte Bedeutung besitzt, oder

b) mit Formaldehyd und Aldehyden der Formel III oder

c) mit Aldehyden III und Harnstoffen der Formel

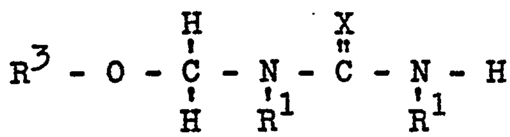 IV,

worin $R^1$ und X die vorgenannte Bedeutung besitzen, und $R^3$ ein Wasserstoffatom oder einen aliphatischen Rest bezeichnet, in einem ersten Schritt bei einer Temperatur unterhalb von 110°C und anschließend in einem zweiten Schritt bei einer Temperatur oberhalb von 110°C in Gegenwart einer Säure umsetzt.

0005504

Europäisches Patentamt

# EUROPÄISCHER RECHERCHENBERICHT

Nummer der Anmeldung
EP 79 10 1412

| Kategorie | EINSCHLÄGIGE DOKUMENTE — Kennzeichnung des Dokuments mit Angabe, soweit erforderlich, der maßgeblichen Teile | betrifft Anspruch | KLASSIFIKATION DER ANMELDUNG (Int.Cl.²) |
|---|---|---|---|
| | US - A - 3 190 741 (H. BRANDEIS et al.)<br>* Spalten 1-6 *<br>-- | 1 | C 07 D 239/10 |
| A | DE - A - 1 670 261 (BASF)<br>* Seiten 1-4,6,8,9 *<br>---- | 1 | |

RECHERCHIERTE SACHGEBIETE (Int. Cl.²)
C 07 D 239/10

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

/ Der vorliegende Recherchenbericht wurde für alle Patentansprüche erstellt.

| Recherchenort | Abschlußdatum der Recherche | Prüfer |
|---|---|---|
| Den Haag | 11-07-1979 | FRANCOIS |

EPA form 1503.1 06.78